# EUROPEAN PATENT SPECIFICATION

(11) **EP 1 274 445 B1**
(45) Date of publication and mention of the grant of the patent: **14.09.2005**
(21) Application number: 00983471.4
(22) Date of filing: 28.12.2000
(51) Int. Cl.: A61K 31/706, A61P 33/00, A61P 35/00

(54) **USE OF STEROIDAL ALKALOIDS TO REVERSE MULTIDRUG RESISTANCE**
VERWENDUNG VON STEROID-ALKALOIDDERIVATEN ZUR UMKEHRUNG MULTIPLER MEDIKAMENTENRESISTENZ
UTILISATION D'ALCALOIDES STEROIDIQUES POUR NEUTRALISER LA RESISTANCE A L'ACTION DE PLUSIEURS MEDICAMENTS

(30) Priority: 30.12.1999 IL 13380999
(43) Date of publication of application: 15.01.2003
(73) Proprietor: YEDA RESEARCH AND DEVELOPMENT CO. LTD., Rehovot 76100 (IL)
(72) Inventor: LISCOVITCH, Mordechai, 69345 Tel-Aviv (IL); LAVIE, Yaakov, 20104 Tzurit, Misgav (IL)
(74) Representative: Vossius & Partner
(86) International application number: PCT/IL2000/000866
(87) International publication number: WO 2001/049279

(56) References cited:
- EP-A- 0 020 029
- EP-A- 0 361 485
- WO-A-97/27211
- WO-A-98/58543
- WO-A-99/52534

## Description

### Field of the Invention

The present invention relates to agents that can reverse multidrug resistance and, in particular, to the use of steroidal alkaloids as inhibitors of multidrug resistance in cancer, and in bacterial, fungal and parasitic infections.

### Background of the Invention

Cancer chemotherapy employs a range of cytotoxic drugs that target rapidly dividing cells and is a major treatment modality in the clinical management of the disease. However, although chemotherapy improves long term survival in cancer patients, it is severely limited by the fact that some forms of cancer are intrinsically refractory to chemotherapeutic agents. Furthermore, chemotherapy often results in subsequent development of tumors that are resistant to most cytotoxic drugs commonly used, leading to an untreatable and incurable disease. It has been estimated that, because of intrinsic or acquired drug resistance, only about 10% of patients that undergo systemic chemotherapy can be cured. In addition, clinical antibacterial, antifungal and antiparasitic treatment may result in bacterial, fungal or parasitic resistance to the drugs.

In the most common form of drug resistance, tumor cells either have or acquire resistance to multiple structurally and functionally unrelated drugs. This phenomenon, termed multidrug resistance (MDR), is often caused by overexpression in the multidrug resistant tumor cells of a plasma membrane ATPase called P-glycoprotein (P-gp) (Ling, 1995). P-gp is a 170-kDa, membrane-bound glycoprotein which is the product of the MDR1 gene. P-gp acts as an energy-dependent drug-efflux pump, increasing outward transport of active drugs and thereby decreasing their intracellular concentration and reducing their cytotoxic efficacy. In addition to P-gp, other drug transporters were identified that are overexpressed in various drug-resistant tumor cell lines. Multidrug resistance protein (MRP) (Cole *et al.,* 1992), is associated with a multidrug resistance phenotype in a number of tumor cell lines that do not overexpress MDR1/P-gp. A homologue of MRP which is localized to the apical membrane of polarized cells has been cloned and termed MRP2 (Keppler and Konig, 1997). MRP2 was recently shown to be involved in conferring MDR (Koike et al., 1997). Other recently discovered MDR proteins are the lung resistance-associated protein (LRP) (Borst et al., 1997) and breast cancer resistance protein (BCRP). P-gp, MRP and BCRP as well as bacterial and fungal MDR proteins belong to the ATP-binding cassette (ABC) protein superfamily of drug transporters, and despite having a different substrate specificity, all seem to operate by facilitating efflux of chemotherapeutic drugs or their conjugates.

It is now clear, however, that P-gp and related ABC transporters are not the sole determinants of drug resistance. MDR cells seem to have adopted multiple strategies in order to survive the lethal effects of chemotherapeutic drugs, and the various cellular mechanisms that contribute to the existence and degree of cross-resistance displayed by MDR cells are suggested to act simultaneously. These additional mechanisms include altered cellular pharmacokinetics of drug uptake, increased metabolic inactivation of drugs, increased DNA repair via alterations in DNA topoisomerase-II activity and loss of programmed cell death. Together with the accelerated outward transport of drugs, mediated by ABC transporters, these mechanisms result in a major decrease in intracellular drug accumulation and effectiveness.

Inhibition of multidrug resistance proteins could be of value in reversing the MDR phenotype. A number of compounds that have little or no cytotoxic action of their own, but inhibit P-gp or MRP-mediated drug export, are capable of sensitizing MDR cells to the cytotoxic effects of chemotherapeutic drugs, thus enabling MDR cell killing. Such compounds are variously called chemosensitizers, MDR modulators, or MDR reversal agents. Known chemosensitizers include compounds of diverse structure and function such as calcium channel blockers (e.g. verapamil), immunosuppressants (e.g. cyclosporine A), antibiotics (e.g. erythromycin), antimalarials (e.g. quinine), phenothiazines (e.g. fluphenazine) and kinase inhibitors (e.g. GF120918) (Hegewisch-Becker, 1996). A few 'first-generation' MDR reversal agents (e.g. verapamil, cyclosporine A) have undergone clinical trials but have not been found to be effective at tolerable doses. Recently, phase II clinical trials with a 'second generation' chemosensitizer (PSC 833) yielded generally positive results and this drug is currently being tested in a phase III trial.

Steroidal alkaloids are plant-derived nitrogen-containing compounds having a 21-, 24- or 27-carbon heterocyclic skeleton. C27 alkaloids derive mainly from the Solanaceae and the Liliaceae, and belong in three major structural groups: solanidanes, spirosolanes and jervanes. Solanidanes and spirosolanes are true steroids, whereas in jervanes the rings are rearranged to form a C-nor-D-homo-steroid (Bruneton, 1995). Appendix A herein depicts the structure of three representative steroidal alkaloids: cyclopamine (a jervane), tomatidine (a spirosolane) and solanidine (a solanidane). Another family of C-nor-D-homo-steroids comprises veratramine in which the E-ring is open (see formula in Appendix B herein).

Steroidal alkaloids are biologically active. Some steroidal alkaloids have teratogenic activity. Cyclopamine, a major steroidal alkaloid of Veratrum californicum, is a potent teratogen when administered at a specific embryonic stage (Keeler, 1978). Solanidine, a steroidal alkaloid which is highly enriched in sprouts of potato (Solanum tuberosum) is less effective as a teratogen, while the tomato (Lycopersicon esculentum) alkaloid tomatidine has no teratogenic activity (Gaffield and Keeler, 1996).

EP 0 020 029 concerns steroidal alkaloids, pharmaceutical compositions comprising the steroidal alkaloids, a process for their extraction from plant and the use thereof as a chemotherapeutic agent.

WO 99/52534 concerns the use of steroidal alkaloid derivatives as inhibitors of hedgehog signaling pathways and pharmaceutical compositions containing the steroidal alkaloid. Further, this publication describes the use of the steroidal alkaloid derivatives for the treatment of neoplastic or hyperplastic transformation.

WO 98/58543 concerns the use of steroid like glycoalkaloids derived from plants for enhancing the innate immunity defense mechanism in vertebrate animals.

EP 361 485 concerns the use of phenothiazines and thioxanthenes (antipsychotic agents) for sensitizing MDR cells to antitumor agents.

WO 97/27211 concerns steroidal carbamates, pharmaceutical composition comprising them and the use thereof for sentisizing MDR cancer cells.

While many functional studies have attributed a wide range of biochemical and pharmacological properties to various steroidal alkaloids, there are no previous reports suggesting that any of these compounds may inhibit MDR. It has now been unexpectedly found that functionally unrelated steroidal alkaloids share the common property of MDR reversal in cancer cells.

It is a purpose of this invention to provide steroidal alkaloids for use as medicaments for inhibiting or reversing MDR in cancer or in bacterial, fungal or parasitic infections.

It is a further purpose of the invention to provide steroidal alkaloids for use in pharmaceutical compositions for treating MDR in cancer or in bacterial, fungal or parasitic infections.

It is yet another object of the invention to provide steroidal alkaloids for use together with anticancer, antibacterial, antifungal or antiparasitic drugs for the preparation of combination treatment modalities.

Other objects and advantages of the invention will become apparent as the description proceeds.

### SUMMARY OF THE INVENTION

It has now been surprisingly found, and this is an object of the invention, that steroidal alkaloids may be used to inhibit or reverse multidrug resistance in human cancer cells. Similar to multidrug resistance in cancer cells, bacterial, fungal and parasitic multidrug resistance is also mediated by the ABC protein superfamily of drug transporters.

The invention is primarily directed to use of at least one steroidal alkaloid or a pharmaceutically acceptable salt thereof in the preparation of a medicament for inhibiting or reversing multi-drug resistance in cancer or in bacterial, fungal or parasitic infections.

It is not intended that the invention, in any of the embodiments described herein, be restricted to any specific compound, but rather to the group of steroidal alkaloids as a class. The steroidal alkaloid may be a natural plant steroidal alkaloid, either extracted from the plant or prepared by chemical synthesis, or a synthetic derivative thereof with modifications in the steroidal backbone such as C-nor-D-homo steroids and/or in the non-steroidal part of the molecule. Examples of such alkaloidal steroids include, but are not limited to, solanidanes, spirosolanes jervanes and veratramines.

In one preferred embodiment of the invention, the steroidal compound used is a spirosolane and is preferably tomatidine (tomatine).

In another more preferred embodiment of the invention, the steroidal alkaloid is a C-nor-D-homo-steroid such as, for example, of the jervane family e.g. cyclopamine and derivatives thereof such as N-methylcyclopamine, cyclopamine-4en-3-one, jervine, tetrahydrojervine and 3-O-acetyljervine. The formulas of these jervane alkaloidal steroids are presented in Appendix B herein.

In another preferred embodiment of the invention, the steroidal alkaloid is a C-nor-D-homo-steroid of the veratramine family in which the E ring is open such as veratramine and dihydroveratramine (Appendix B).

Other examples of steroidal alkaloids that may be used according to the invention include, but are not limited to, (+) verabenzoamine, 15-O-(2-methylbutyroyl)germine, 20-isoveratramine, angeloylzygadenine, germerine, germanitrine, germidine, germine, maackinine, neogermbudine, peimisine, rubijervine, solanidine (solanine), solanocapsine, solasodine (solasonine), veralkamine, verapatuline, veratrine (extract mixture), veratrosine, verazine, verazinine, vertaline, verticine, verussurine, verusurinine and zygadenine.

The invention also provides a steroidal alkaloid or a pharmaceutically acceptable salt thereof for use as a medicament in the treatment of multidrug resistance in cancer or in bacterial, fungal or parasitic infections.

In another aspect, the invention is directed to the use of a steroidal alkaloid and an agent selected from an anticancer, antibacterial, antifungal or antiparasitic agent in the preparation of a medicament for the treatment of multidrug resistance in cancer or in bacterial, fungal or parasitic infections.

The invention is further directed to the use of a combination of a steroidal alkaloid and an anticancer, antibacterial, antifungal or antiparasitic agent in the preparation of a medicament for the treatment of multidrug resistant cancers or bacterial, fungal or parasitic infections.

The invention further describes a pharmaceutical composition for the treatment of multidrug resistance comprising as an active ingredient one or more steroidal alkaloids or pharmaceutically acceptable salts thereof, together with one or more pharmaceutically acceptable carriers, excipients or diluents.

The invention also describes a pharmaceutical composition for treatment of multidrug resistant cancers or bacterial, fungal or parasitic infections, comprising as active ingredients a steroidal alkaloid and an anticancer, antibacterial, antifungal or antiparasitic agent, respectively, or pharmaceutically acceptable salts thereof, together with one or more pharmaceutically acceptable carriers, excipients or diluents.

The pharmaceutical compositions according to the invention will be formulated in a form similar to any steroidal treatment, such as for oral administration in the form of capsules, tablets and the like, as emulsions or as solutions suitable for infusion or injection, particularly for intramuscular injection. The amounts of the steroidal alkaloid in the composition will depend on the type and stage of the disease and the condition and age of the patient and will be determined by the physician skilled in the art.

The invention further relates to the use of an effective amount of a steroidal alkaloid for the preparation of a medicament for the inhibition of development of multidrug resistance or reversal of multidrug resistance developed in a cancer patient after undergoing chemotherapy. The steroidal alkaloid will inhibit the development of multidrug resistance and reduce drug-resistance in drug-resistant tumors, thus potentiating the effect of antineoplastic drugs.

According to this aspect of the invention, the steroidal alkaloid is suitable to be administered to the cancer patient in combination with the anticancer agent, either prior to or simultaneously with the suitable anticancer agent. Any antineoplastic agent may be used in combination with the steroidal alkaloid according to the type of tumor and the chosen chemotherapy protocol. Examples of such antineoplastic agents include, but are not limited to, adriamycin, methotrexate, taxol, 5-fluorouracyl, vinblastine, vincristine, mitomycin, cisplatin and the like.

The invention still further relates to the use of an effective amount of a steroidal alkaloid, optionally together with an antibacterial, antifungal or antiparasitic agent for the preparation of a medicament for the reversal of multidrug resistance developed in a patient suffering from a bacterial, fungal or parasitic infection after undergoing antibiotic or other therapy. The steroidal alkaloid ill inhibit the development of drug resistance to the bacteria, fungi or parasites and reduce drug-resistance of drug-resistant pathogenic organisms, thus potentiating the effect of antibacterial, antifungal or antiparasitic drugs. The steroidal alkaloid may be administered prior to or simultaneously with the antibacterial, antifungal or antiparasitic agent.

All the above and other characteristics and advantages of the invention will be further understood from the following illustrative and non-limitative examples of preferred embodiments thereof.

### Brief Description of the Drawings

The present invention will be more clearly understood from the detailed description of the preferred embodiments and from the attached drawings in which:

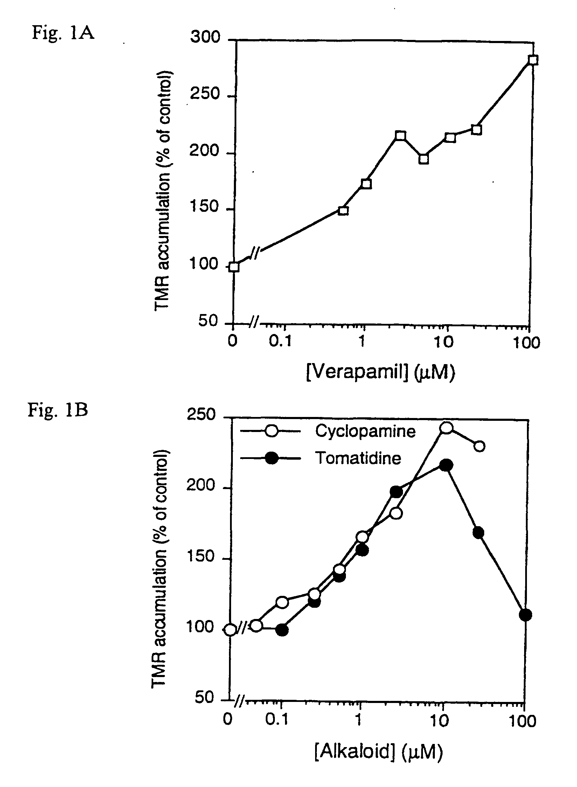

Figs. 1A-1B show that cyclopamine and tomatidine enhance tetramethylrosamine chloride (TMR) accumulation in multidrug resistant MCF-7-AdrR breast cancer cells. TMR accumulation was determined as described in Example 1 in MCF-7-AdrR cells treated with the indicated concentrations of verapamil (Fig. 1A), cyclopamine or tomatidine (Fig. 1B). The results are expressed as the mean of triplicate determinations from a representative experiment.

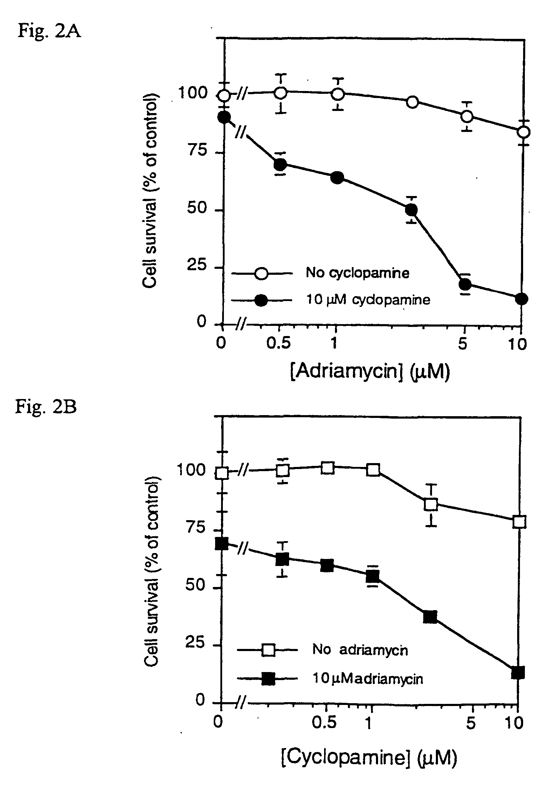

Figs. 2A-2B show that cyclopamine sensitizes MCF-7-AdrR cells to the cytotoxic action of adriamycin. MCF-7-AdrR cells were treated with increasing concentrations of adriamycin (Fig. 2A) and cyclopamine (Fig. 2B) in the absence or presence of 10 µM cyclopamine or adriamycin, respectively. Cell survival was determined after 48 hours of incubation using the MTT assay, as described in Example 2. The results are expressed as percentage of control, drug-free wells and represent the mean±S.D. of quadruplicate determinations from a representative experiment that was repeated at least twice.

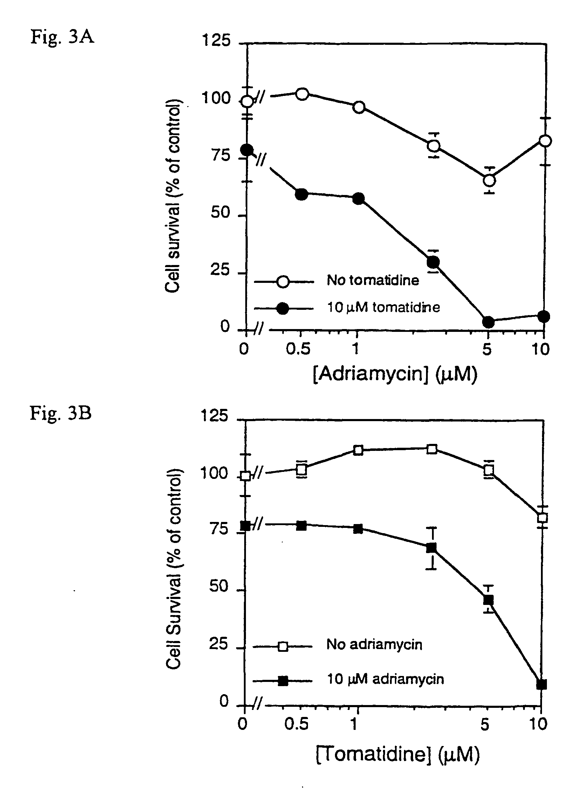

Figs. 3A-3B show that tomatidine sensitizes MCF-7-AdrR cells to the cytotoxic action of adriamycin. MCF-7-AdrR cells were treated with increasing concentrations of adriamycin (Fig. 3A) and tomatidine (Fig. 3B) in the absence or presence of 10 µM tomatidine or adriamycin, respectively. Cell survival was determined after 48 hours of incubation using the MTT assay, as described in Example 2. The results are expressed as percentage of control, drug-free wells and represent the mean±S.D. of quadruplicate determinations from a representative experiment that was repeated at least twice.

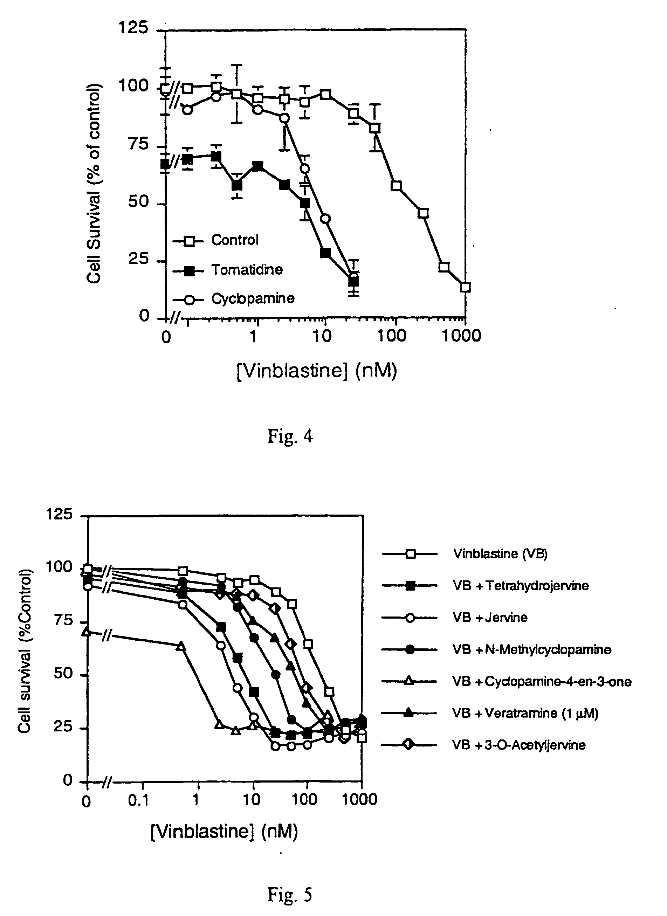

Fig. 4 shows that cyclopamine and tomatidine sensitize MCF-7-AdrR cells to the cytotoxic action of vinblastine. MCF-7-AdrR cells were treated with increasing concentrations of vinblastine in the absence or presence of 10 µM cyclopamine or tomatidine. Cell survival was determined after 48 hours of incubation using the MTT assay, as described in Example 2. The results are expressed as percentage of control, drug-free wells and represent the mean±S.D. of quadruplicate determinations from a representative experiment that was repeated at least twice.
Fig. 5 shows that jervane steroidal alkaloids and veratramine sensitize MCF-7-AdrR cells to the cytotoxic action of vinblastine. MCF-7-AdrR cells were treated with increasing concentrations of vinblastine in the absence or presence of 10 µM jervane steroidal alkaloid or 1 µM veratramine. Cell survival was determined after 48 hours of incubation using the MTT assay, as described in Example 2. The results are expressed as percentage of control, drug-free wells and represent the mean of quadruplicate determinations from a representative experiment that was repeated at least twice.

### Detailed Description of Preferred Embodiments

For purposes of clarity and as an aid in the understanding of the invention, as disclosed and claimed herein, the following terms and abbreviations are defined below:
**Steroidal alkaloid -** as herein comprises any natural plant steroidal alkaloid either extracted from the plant, or prepared by chemical synthesis, as well as synthetic derivatives thereof with modifications in the steroidal backbone, e.g. C-nor-D-homo steroids, and/or in the non-steroidal part of the molecule.
**MDR -** multidrug resistance in cancers, as well as in bacterial, fungal and parasitic infections.

Although it is not intended that the invention be limited or restricted to any one steroidal alkaloid, or structural class of alkaloids, the following table gives illustrative examples of some of the compounds included within the scope of the invention. It is to be emphasized that this list is for the purpose of illustration and example only, and does not limit the invention in any way:

| **Partial list of steroidal alkaloids** | |
|---|---|
| **Name** | **Plant source** |
| (+)- Verabenzoamine | *Veratrum nigrum* |
| 15-O-(2-Methylbutyroyl)germine | |
| 20-Isoveratramine | *Veratrum patulum* |
| Angeloylzygadenine | |
| Cyclopamine | *Veratrum californicum* |
| Germanitrine | |
| Germerine | |
| Germidine | |
| Germine | *Veratrum sp.* |
| Jervine | *Veratrum californicum* |
| Maackinine | *Veratrum maackii* |
| Neogermbudine | |
| Peimisine | *Fritillaria siechuanica* |
| Rubijervine | *Veratrum sp.* |
| Solanidine (solanine) | *Solanum tuberosum* |
| Solanocapsine | |
| Solasodine (solasonine) | *Solanum melongata* |
| Tomatidine (tomatine) | *Lycopersicon esculentum* |
| Veralkamine | *Veratrum album* |
| Verapatuline | *Veratrum patulum* |
| Veratramine | *Veratrum grandiflorum; V. viride* |
| Veratrine (extract; mixture) | |
| Veratrosine | |
| Verazine | |
| Verazinine | |
| Vertaline | *Veratrum taliense* |
| Verticine | *Fritillaria verticillata* |
| Verussurine | *Veratrum nigrum* |
| Verusurinine | *Veratrum nigrum* |
| Zygadenine | *Zygadenus sp.* |

Cyclopamine and other Veratrum steroidal alkaloids may be obtained as described by Keeler, R.F. (1969, Phytochemistry 8: 223); solanidine and other Solanum steroidal alkaloids as described by Gaffield, V. And Keeler, R.F. (1996, Chem. Res. Toxicol. 9: 426-433), and tomatidine and other Lycopersicon steroidal alkaloids as described by Nagoka *et al*. (1993, Phytochemistry 34: 1153-57).

The alkaloidal steroids of the invention may be administered alone, but in general they will be prepared as admixtures with pharmaceutically acceptable carriers, diluents or excipients. The selection and use of these components will be made with reference to the desired route of administration, and in accordance with standard pharmaceutical practice. When intended for oral administration, for example, they may be prepared as tablets containing excipients such as starch or lactose. Alternatively, the compounds of the invention may be formulated as capsules, either with or without the addition of the aforementioned excipients. The compounds may also be prepared as syrups, elixirs or suspensions containing suitable colouring, flavouring and thickening agents. For the purposes of parenteral administration (e.g. by the intravenous, intramuscular, subcutaneous or intradermal routes), the preferred route in the treatment of cancer, the compounds may be prepared as sterile aqueous solutions which may also contain salts, sugars etc., for the purpose of achieving isotonicity. The alkaloids of the invention may also be prepared for topical administration in the form of solutions, ointments, creams, salves and the like, by the addition of appropriate carriers, stabilis and thickeners. Each of the foregoing types of preparation may be used for the preparation of both pharmaceutical compositions containing the steroidal alkaloids alone, or as combination preparations together with other agents.

The following examples are brought to illustrate the activities of the compounds of the invention as inhibitors of multidrug resistance in cancer cells.

### Examples

### Materials:

Cyclopamine and other jervane steroids were kindly provided by Dr. William Gaffield (Western Regional Research Center, Albany, CA). Veratramine is commercially available. Tomatidine, solanidine, solasodine, adriamycin, vinblastine, verapamil and (3-[4,5-dimethylthyazol-2-yl]-2,5-diphenyltetrazolium bromide (MTT) were purchased from Sigma (St. Louis, MO). Tetramethylrosamine chloride (TMR) was purchased from Molecular Probes (Eugene, OR). Fetal calf serum (FCS) and tissue culture grade antibiotics were obtained from Biological Industries (Beth Haemek, Israel).

### Cell culture:

MCF-7 human breast adenocarcinoma cells and adriamycin-resistant MCF-7-AdrR cells were kindly provided by Dr. Merrill E. Goldsmith (National Cancer Institute, Bethesda, MD). MCF-7 and derived cell lines were grown according to published procedures (Fairchild, C.R. *et al*., Mol. Pharmacol. 37: 801-809, 1990).

### Example 1

### Effect of steroidal alkaloids on in vitro drug uptake model

The effect of steroidal alkaloids on drug transport was examined by measuring cellular accumulation of TMR, a fluorescent model drug, according to Eytan et al. (Eytan, G.D. *et al*., Eur. J. Biochem. 248: 104-112, 1997) with some modifications. Cells were seeded in 24-well tissue culture plates at a density of 0.5 X 10⁵ cells/well and were grown for 24 h in growth medium supplemented with 0.5% FCS. TMR (diluted from a 10 mM stock in DMSO) was added to the cells at a final concentration of 10 µM, in 0.5 ml of growth medium, in the presence or absence of the tested alkaloids (given prior to the dye as indicated). Cells were incubated with TMR and the tested drug for 30 minutes at 37°C. To terminate the assay, the plates were placed on ice and the cells were washed three times with ice-cold phosphate-buffered saline (PBS) in order to remove residual TMR. The cells were then lysed by incubation with 0.5 ml of 0.5 N NaOH for 20 minutes at room temperature, and the lysates were neutralized with 0.5 ml of 0.5 N HCl and collected. Cell-associated TMR was determined by measuring the fluorescence intensity of the lysates using a fluorescence spectrophotometer (excitation at 555-nm, emission at 575-nm). Blank values obtained at zero time were subtracted from all the fluorescence values and the results were normalized to the amount of cellular protein in each well. Experiments were carried out in triplicates and were repeated at least twice. Results are expressed as a percentage of incubations with TMR alone.

Expression of multidrug transporters markedly decreases drug accumulation in MDR cells. Conversely, in such cells drug accumulation is increased by inhibition of P-gp and related proteins with MDR reversal agents. TMR accumulation was utilized to examine the effect of steroidal alkaloids on P.gp in MCF-7-AdrR adriamycin-resistant human breast adenocarcinoma cells. Preliminary experiments demonstrated that incubation of MCF-7-AdrR cells with TMR resulted in accumulation of TMR in the cells. TMR accumulation in MCF-7-AdrR cells is 3-to 6-fold lower than that of the parental MCF-7 cells (data not shown). MDR reversal agents (e.g. verapamil) elevated TMR accumulation back to near normal levels (Fig. 1A). Cyclopamine and tomatidine caused a concentration-dependent increase of TMR accumulation in the MCF-7-AdrR cells (Fig. 1B). TMR accumulation was maximally stimulated by cyclopamine and tomatidine by 2- and 2.5-fold, respectively, and was half maximally effective at a concentration of about 1 µM.

### Example 2

### Effect of steroidal alkaloids on in vitro Cytotoxicity assay

Cells were plated in 96-well plates at a density of 4 X 10³ cells/well in 0.1 ml drug-free DMEM containing 5% fetal calf serum, and incubated at 37°C for 48 h. After this time, cytotoxic drugs (adriamycin or vinblastine) were added to the wells at the indicated concentrations, in the absence or presence of the tested steroidal alkaloids, and the cells were further incubated for an additional 48-72 h. The cytotoxic activity of the drugs was then determined using a standard MTT cell survival assay (Hansen, M.B. *et al*., J. Immunol. Meth. 119: 203-210. The MTT reagent (diluted from a 5 mg/ml solution in PBS) was added to all the wells at a final concentration of 0.6 mg/ml and the cells were further incubated at 37°C for 2 or 3 h. The reaction was terminated by adding 100 µl/well of an extraction solution consisting of 20% (w/v) sodium dodecyl sulfate (SDS) in 50% aqueous dimethyl formamide solution, pH 4.8. The plates were left overnight at room temperature in the dark, following which the absorbance was read at 570-nm using an ELISA plate reader. Three to six wells were treated with 1% SDS (final concentration) for 5 minutes prior to adding the MTT reagent, and the average absorbance values obtained from these wells served as blank and was subtracted from all other results. Data points represent the mean±S.D. of a quadruplicate determination from a representative experiment that was repeated at least twice. The results are expressed as a percentage of control, drug-free wells.

The effect of the steroidal alkaloids on TMR uptake indicated that they could act to increase the cytotoxic effects of drugs, such as adriamycin and vinblastine, on MDR cancer cells. The cytotoxic effect of the drugs was evaluated by utilizing the MTT cell viability assay, a standard assay for assessing drug resistance and its reversal. Routinely, MCF-7-AdrR cells were exposed to increasing concentrations of drugs for 48 h and the number of viable cells was quantitated after adding the MTT reagent. Preliminary experiments have confirmed that MCF-7-AdrR cells are significantly less sensitive to adriamycin as compared to the parental MCF-7 cells, and that MDR reversal agents (e.g. verapamil) markedly increase their drug sensitivity (data not shown). At a maximal concentration of 10 µM, adriamycin reduced MCF-7-AdrR cell survival by no more than 20-25% (Fig. 2A; open circles). In contrast, incubation of the cells with increasing concentrations of adriamycin in the presence of a fixed concentration of cyclopamine (10 µM) resulted in a dose-dependent, nearly 90% reduction of cell viability, compared with drug-free incubations (Fig. 2A; solid circles). Cyclopamine alone reduced cell survival by 10-20%. The dependence of the chemosensitizing effect of cyclopamine on its concentration was determined by incubating the cells with a fixed concentration of adriamycin (10 µM) in the presence of increasing concentrations of cyclopamine. The effect of cyclopamine was concentration-dependent. At this concentration of adriamycin, cyclopamine sensitized the cells to adriamycin with an EC₅₀ of 2.5 µM (Fig. 2B).

A similar set of experiments was carried out with the spirosolane alkaloid tomatidine. As shown in Fig. 3, adriamycin alone was relatively ineffective even at the maximal tested concentration of 10 µM. Conversely, in the presence of tomatidine (10 µM), cell viability was reduced by more than 90%. Tomatidine itself had a mild cytotoxic effect on the cells, reducing cell viability by 20-25% at a concentration of 10 µM. The sensitizing effect of tomatidine on adriamycin toxicity was concentration-dependent (Fig. 3A). At an adriamycin concentration of 10 µM, tomatidine sensitized the cells with an EC₅₀ of 5 µM (Fig. 3B).

Site-directed mutagenesis studies have indicated that transport of different drugs may be differentially affected by specific mutations, suggesting that there is more than one drug-interaction site on the P-gp molecule. As opposed to adriamycin, which is a topoisomerase II inhibitor, vinblastine is a tubulin-active antimitotic agent that appears to interact with a different site on P-gp. It was therefore important to examine the effect of steroidal alkaloids on the resistance of MCF-7-AdrR to vinblastine. Vinblastine caused a nearly complete, concentration-dependent reduction of cell viability, with an LD₅₀ of 200 nM (Fig. 4). Cyclopamine (10 µM) markedly shifted the vinblastine concentration- response curve to the left, resulting in a LD₅₀ value for vinblastine of 8 nM. Similar results were seen with the spirosolane alkaloid tomatidine except that, as seen above, it had a modest cytotoxic activity of its own and it shifted vinblastine to a LD₅₀ value of 5 nM. These results indicate that steroidal alkaloids can sensitize MDR cells to structurally and functionally diverse cytotoxic drugs.

A number of additional Jervane family members and veratramine (see Appendix B herein) were tested as multidrug resistance chemosensitizers. The response of multidrug resistant MCF-7/AdrR breast adenocarcinoma cells to the tested agents was examined by utilizing the MTT cell viability assay. Thus, MCF-7/AdrR cell survival was tested after exposure to increasing concentrations of vinblastine in the absence or in the presence of a fixed concentration of the tested compounds (all at 10 µM except veratramine, 1µM) as indicated in Fig. 5. The results show that all C-*nor*-D-*homo*-steroids tested were effective in shifting the vinblastine concentration-response curve to the left and that structural modifications of the C-*nor*-D-*homo*-steroid backbone can modify the pharmacological activity of the compound.. The most effective compounds were cylopamine-4-en-3-one (blank triangles), jervine (blank circles) and tetrahydrojervine (black/white squares). The results indicate that steroidal alkaloids having a C-nor-D-homo-steroid tetracyclic backbone have the ability to sensitize multidrug resistant cancer cells to the cytotoxic actions of chemotherapeutic drugs and may thus serve as MDR reversal agents in combination cancer chemotherapy.

### Example 3

### In vivo MDR assay

After establishing *in vitro* activity of a compound as an MDR modulator, it is essential to evaluate its reversal efficiency in a tumor-bearing animal model. This assay is carried out according to Watanabe *et al*. (Anti-Cancer Drugs 7: 825-832, 1996). P388-VCR cells (10⁶) are inoculated by intraperitoneal injections (0.1 ml of saline) in the BALB/C X DBA/2 (CDF₁) mice on day 0. The P388-VCR bearing mice (30 mice in each group) are treated with control vehicle, adriamycin, tested steroidal alkaloid or combinations of these on days 1, 5 and 9. Tested compounds are administered 1 hour prior to treatment with adriamycin. Survival of mice in each group is examined daily. Anti-tumor activity is evaluated based on (1) mean survival time of the drug (adriamycin)-treated mouse group (T) divided by the mean survival time of the control group (C) [T/C (%)] and (2) mean survival time of tested compound-treated mouse group (A) divided by the mean survival time of adriamycin-treated group (T) [A/T (%)]. The experiment is repeated at least three times.

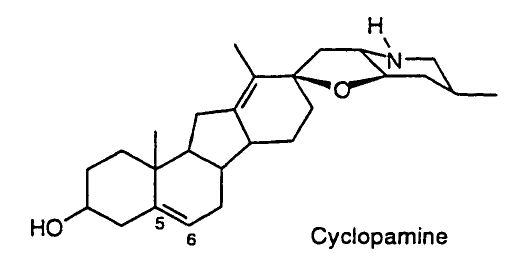

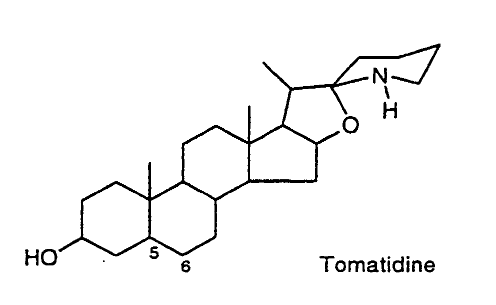

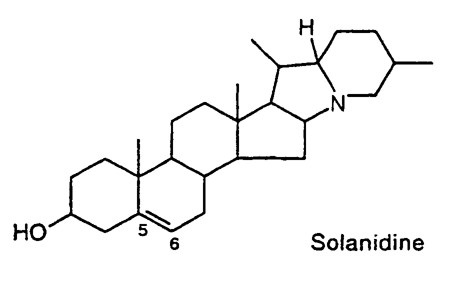

### Appendix A

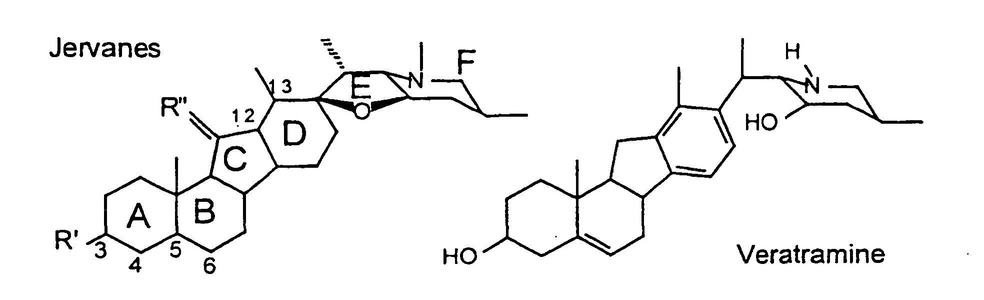

| Jervane | R' | R" | C4-5 | C5-6 | C12-13 | N |
|---|---|---|---|---|---|---|
| Cyclopamine | OH | H2 | - | = | = | H |
| *N*-Methylcyclopamine | OH | H2 - | | = | = | CH3 |
| Cyclopamine-4-en-3-one | O= | H2 | = | - | = | H |
| Jervine | OH | O= | - | = | = | H |
| Tetrahydrojervine | OH | O= | - | - | - | H |
| 3-O-Acetyljervine | O-Ac | O= | - | - | = | H |

### Appendix B

### References

Bruneton, J. (1995) Terpenoid alkaloids, in "Pharmacognosy, Phytochemistry, Medicinal Plants", ed., Intercept Ltd., pp. 863-878.

Cole, S.P.C., Bhardwaj, G., Gerlach, J.H., Mackie, J.E., Grant, C.E., Almquist, K.C., Stewart, A.J., Kurz, E.U., Duncan, A.M. and Deeley, R.G. (1992) Overexpression of a transporter gene in a multidrug-resistant human lung cancer cell line. Science 258: 1650-1654.

Gaffield, W. and Keeler, R.F. (1996) Induction of terata in hamsters by solanidane alkaloids derived from Solanum tuberosum. Chem. Res. Toxicol. 9: 426-433.

Hegewisch-Becker, S. (1996) MDR1 reversal: criteria for clinical trials designed to overcome the multidrug resistance phenotype. Leukemia 10 (Suppl. 3) S32-S38.

Keeler, R.F. (1978) Cyclopamine and related steroidal alkaloid teratogens: Their occurrence, structural relationship, and biologic effects. Lipids 13: 708-715.

Keppler, D. and Konig, J. (1997) Expression and localization of the conjugate export pump encoded by the MRP2 (cMRP/cMOAT) gene in liver. FASEB J. 11: 509-516.

Koike, K., Tawabe, T., Tanaka, T., Toh, S., Uchiumi, T., Wada, M., Akyiama, S., Ono, M., and Kuwano, M. (1997) A canalicular multispecific organic anion transporter (cMOAT) antisense cDNA enhances drug sensitivity in human hepatic cancer cells. Cancer Res. 57: 5475-79.

Ling, V. (1995) P-glycoprotein: Its role in multidrug resistance. Am. J. Med. 99 (6A): 31S - 34S.

## Claims

1. Use of a steroidal alkaloid or a pharmaceutically acceptable salt thereof for the preparation of a medicament for inhibiting or reversing multidrug resistance in cancer or in bacterial, fungal or parasitic infections.

2. Use of one or more steroidal alkaloids or pharmaceutically acceptable salts thereof for the preparation of a pharmaceutical composition for the inhibition or treatment of multidrug resistance in cancer or in bacterial, fungal or parasitic infections.

3. Use of a steroidal alkaloid or pharmaceutically acceptable salt thereof for the preparation of a pharmaceutical composition for the inhibition of development of drug resistance or for reversal of multidrug resistance in a patient suffering from cancer or from bacterial, fungal or parasitic infection.

4. The use of any one of claims 1 to 3, wherein said steroidal alkaloid is a natural plant steroidal alkaloid or a synthetic derivative thereof.

5. The use of any one of claims 1 to 4, wherein said steroidal alkaloid is of the solanidane or spirosolane family.

6. The use of any one of claims 1 to 5, wherein said steroidal alkaloid is a spirosolane.

7. The use of claim 6, wherein said spirosolane is tomatidine.

8. The use of any one of claims 1 to 4, wherein said steroidal alkaloid is a C-nor-D-homo steroid.

9. The use of any one of claims 1 to 4 or 8, wherein said steroidal alkaloid is of the jervane or veratramine family.

10. The use of claim 9, wherein said jervane is selected from the group consisting of: cyclopamine, cyclopamine-4en-3-one, jervine and tetrahydrojervine.

11. The use of claim 9, wherein said jervane is selected from the group consisting of: N-methylcyclopamine and 3-O-acetyljervine.

12. The use of any one of claims 1 to 4 or 9, wherein said steroidal alkaloid is veratramine.

13. The use of any one of claims 1 to 4, wherein said steroidal alkaloid is selected from the group consisting of: (+) verbenzoamine, 15-O-(2-methylbutyroyl)germine, 20-isoveratramine, angeloylzygadenine, germerine, germanitrine, germidine, germine, maackinine, neogermbudine, peimisine, rubijervine, solanidine (solanine), solanocapsine, solasodine (solasonine), veralkamine, verapatuline, veratrine (extract mixture), veratrosine, verazine, verazinine, vertaline, verticine, verussurine, verusurinine and zygadenine.

14. The use of any one of claims 1 to 13, wherein said medicament or pharmaceutical composition is for inhibiting or reversing multidrug resistance in cancer.

15. The use of claim 14, wherein said medicament or pharmaceutical composition further comprises an anti-cancer agent or wherein said pharmaceutical composition is administered in combination with an anti-cancer agent.

16. The use of claim 15, wherein said anti-cancer agent is adriamycin, methotrexate, taxol, 5-fluorouracyl, vinblastine, vincristine, mitomycin, or cisplatin.

17. The use of any one of claims 1 to 13, wherein said medicament or pharmaceutical composition is for inhibiting or reversing multidrug resistance in bacterial, fungal or parasitic infections.

18. The use of claim 17, wherein said medicament or pharmaceutical composition further comprises or is administered in combination with an agent selected from an antibacterial, antifungal or antiparasitic agent.

19. The use of claim 15 or 16, wherein said medicament or pharmaceutical composition is administered prior to, or simultaneously with, the anticancer agent.

## Patentansprüche

1. Verwendung eines Steroid-Alkaloids oder eines pharmazeutisch verträglichen Salzes davon für die Herstellung eines Medikaments zur Hemmung oder Umkehrung von multipler Medikamentenresistenz bei Krebserkrankungen oder bakteriellen, mykotischen oder parasitären Infektionen.

2. Verwendung eines oder mehrerer Steroid-Alkaloide oder pharmazeutisch verträglicher Salze davon für die Herstellung eines Arzneimittels zur Hemmung oder Behandlung von multipler Medikamentenresistenz bei Krebserkrankungen oder bakteriellen, mykotischen oder parasitären Infektionen.

3. Verwendung eines Steroid-Alkaloids oder eines pharmazeutisch verträglichen Salzes davon für die Herstellung eines Arzneimittels zur Hemmung der Ausbildung einer Medikamentenresistenz oder zur Umkehrung von multipler Medikamentenresistenz bei einem Patienten, der an einer Krebserkrankung oder einer bakteriellen, mykotischen oder parasitären Infektion leidet.

4. Verwendung gemäß einem der Ansprüche 1 bis 3, wobei das Steroid-Alkaloid ein Steroid-Alkaloid aus einer natürlichen Pflanze oder ein synthetisches Derivat davon ist.

5. Verwendung gemäß einem der Ansprüche 1 bis 4, wobei das Steroid-Alkaloid aus der Familie der Solanidane oder Spirosolane stammt.

6. Verwendung gemäß einem der Ansprüche 1 bis 5, wobei das Steroid-Alkaloid ein Spirosolan ist.

7. Verwendung gemäß Anspruch 6, wobei das Spirosolan Tomatidin ist.

8. Verwendung gemäß einem der Ansprüche 1 bis 4, wobei das Steroid-Alkaloid ein C-Nor-D-homo-steroid ist.

9. Verwendung gemäß einem der Ansprüche 1 bis 4 oder 8, wobei das Steroid-Alkaloid aus der Familie der Jervane oder Veratramine stammt.

10. Verwendung gemäß Anspruch 9, wobei das Jervan ausgewählt ist aus Cyclopamin, Cyclopamin-4en-3-on, Jervin und Tetrahydrojervin.

11. Verwendung gemäß Anspruch 9, wobei das Jervan ausgewählt ist aus N-Methylcyclopamin und 3-O-Acetyljervin.

12. Verwendung gemäß einem der Ansprüche 1 bis 4 oder 9, wobei das Steroid-Alkaloid Veratramin ist.

13. Verwendung gemäß einem der Ansprüche 1 bis 4, wobei das Steroid-Alkaloid ausgewählt ist aus (+)-Verbenzoamin, 15-O-(2-Methylbutryoyl)germin, 20-Isoveratramin, Angeloylzygadenin, Germerin, Germanitrin, Germidin, Germin, Maackinin, Neogermbudin, Peimisin, Rubijervin, Solanidin (Solanin), Solanocapsin, Solasodin (Solasonin), Veralkamin, Verapatulin, Veratrin (Extraktgemisch), Veratrosin, Verazin, Verazinin, Vertalin, Verticin, Verussurin, Verusurinin und Zygadenin.

14. Verwendung gemäß einem der Ansprüche 1 bis 13, wobei das Medikament oder Arzneimittel multiple Medikamentenresistenz bei Krebs hemmt oder umkehrt.

15. Verwendung gemäß Anspruch 14, wobei das Medikament oder Arzneimittel weiterhin einen Antikrebswirkstoff umfaßt oder wobei das Arzneimittel in Kombination mit einem Antikrebswirkstoff verabreicht wird.

16. Verwendung gemäß Anspruch 15, wobei der Antikrebswirkstoff Adriamycin, Methotrexat, Taxol, 5-Fluoruracyl, Vinblastin, Vincristin, Mitomycin oder Cisplatin ist.

17. Verwendung gemäß einem der Ansprüche 1 bis 13, wobei das Medikament oder Arzneimittel multiple Medikamentenresistenz bei bakteriellen, mykotischen oder parasitären Infektionen hemmt oder umkehrt.

18. Verwendung gemäß Anspruch 17, wobei das Medikament oder Arzneimittel weiterhin einen Wirkstoff ausgewählt aus einem antibakteriellen, antimykotischen oder antiparasitären Wirkstoff umfaßt oder in Kombination mit diesem verabreicht wird.

19. Verwendung gemäß Anspruch 15 oder 16, wobei das Medikament oder Arzneimittel vor oder gleichzeitig mit dem Antikrebswirkstoff verabreicht wird.

## Revendications

1. Utilisation d'un alcaloïde stéroïde ou un de ses sels pharmaceutiquement acceptable pour la préparation d'un médicament destiné à inhiber ou inverser la résistance à plusieurs drogues dans un cancer ou des infections bactériennes, fongiques ou parasitaires.

2. Utilisation d'un ou plusieurs alcaloïdes stéroïdes ou leurs sels pharmaceutiquement acceptables pour la préparation d'une composition pharmaceutique destinée à inhiber ou à traiter une résistance à plusieurs drogues dans un cancer ou des infections bactériennes, fongiques ou parasitaires.

3. Utilisation d'un alcaloïde stéroïde ou un de ses sels pharmaceutiquement acceptables pour la préparation d'une composition pharmaceutique destinée à inhiber le développement d'une résistance à une drogue ou à inverser une résistance à plusieurs drogues chez un patient atteint d'un cancer ou d'une infection bactérienne, fongique ou parasitaire.

4. Utilisation selon l'une quelconque des revendications 1 à 3, dans laquelle ledit alcaloïde stéroïde est un alcaloïde stéroïde de plante naturelle ou un de ses dérivés synthétiques.

5. Utilisation selon l'une quelconque des revendications 1 à 4, dans laquelle ledit alcaloïde stéroïde est de la famille des solanidane ou spirosolane.

6. Utilisation selon l'une quelconque des revendications 1 à 5, dans laquelle ledit alcaloïde stéroïde est un spirosolane.

7. Utilisation selon la revendication 6, dans laquelle ledit spirosolane est la tomatidine.

8. Utilisation selon l'une quelconque des revendications 1 à 4, dans laquelle ledit alcaloïde stéroïde est un C-nor-D-homo stéroïde.

9. Utilisation selon l'une quelconque des revendications 1 à 4 ou 8, dans laquelle ledit alcaloïde stéroïde est de la famille des jervane ou veratramine.

10. Utilisation selon la revendication 9, dans laquelle ladite jervane est choisie parmi : la cyclopamine, cyclopamine-4-èn-3-one, jervine et tetrahydrojervine.

11. Utilisation selon la revendication 9, dans laquelle ladite jervane est choisie parmi la N-méthylcyclopamine et la 3-O-acétyljervine.

12. Utilisation selon l'une quelconque des revendications 1-4 ou 9, dans laquelle ledit alcaloïde stéroïde est la veratramine.

13. Utilisation selon l'une quelconque des revendications 1-4, dans laquelle ledit alcaloïde stéroïde est choisi parmi : (+)verbenzoamine, 15-O-(2-méthylbutyroyl)germine, 20-isoveratramine, angeloylzygadenine, germerine, germanitrine, germidine, germine, maackinine, neogermbudine, peimisine, rubijervine, solanidine (solanine), solanocapsine, solasodine (solasonine), veralkamine, verapatuline, veratrine (mélange d'extrait), veratrosine, verazine, verazinine, vertaline, verticine, verussurine, verusurinine et zygadenine.

14. Utilisation selon l'une quelconque des revendications 1-13, dans laquelle ledit médicament ou composition pharmaceutique est destiné à inhiber ou inverser la résistance à plusieurs drogues dans un cancer.

15. Utilisation selon la revendication 14, dans laquelle ledit médicament ou composition pharmaceutique comprend en outre un agent anti-cancéreux ou dans laquelle ladite composition pharmaceutique est administrée en combinaison avec un agent anti-cancéreux.

16. Utilisation selon la revendication 15, dans laquelle ledit agent anti-cancéreux est l'adriamycine, le méthotrexate, le taxol, le 5-fluorouracyl, la vinblastine, vincristine, mitomycine ou le cisplatine.

17. Utilisation selon l'une quelconque des revendications 1-13, dans laquelle ledit médicament ou composition pharmaceutique est destiné à inhiber ou inverser la résistance à plusieurs drogues dans des infections bactériennes, fongiques ou parasitaires.

18. Utilisation selon la revendication 17, dans laquelle ledit médicament ou composition pharmaceutique comprend en outre ou est administré en combinaison avec un agent choisi parmi un agent antibactérien, antifongique ou antiparasitaire.

19. Utilisation selon la revendication 15 ou 16, dans laquelle ledit médicament ou composition pharmaceutique est administré avant, ou simultanément à, l'agent anticancéreux.
